# EUROPEAN PATENT APPLICATION

(11) **EP 2 599 517 A1**
(43) Date of publication of application: **05.06.2013**
(21) Application number: 11812575.6
(22) Date of filing: 28.07.2011
(51) Int. Cl.: A61M 35/00, A61K 9/70

(54) **DRUG SUPPORT BODY, AND METHOD FOR PRODUCING SAME**

(30) Priority: 29.07.2010 JP 2010171027
(71) Applicant: Santen Pharmaceutical Co., Ltd, Osaka-shi Osaka 533-8651 (JP); Tsukioka Film Pharma Co., Ltd., Gifu 509-0109 (JP)
(72) Inventor: TASAKA Fumitaka, Ikoma-shi Nara 630-0101 (JP); MIYOSHI Naohito, Ikoma-shi Nara 630-0101 (JP); HORIBE Yoshihide, Ikoma-shi Nara 630-0101 (JP)
(74) Representative: Lemcke, Brommer & Partner
(86) International application number: PCT/JP2011/067291
(87) International publication number: WO 2012/014986

(57) **Abstract**

A drug support body is provided that can be used to appropriately administer a drug to the anterior segment of the eye while the production cost is reduced. A drug layer 2 containing a drug component to be administered to an anterior segment of the eye is provided at a halfway portion in a longitudinal direction of a base plate 3, wherein the base plate 3 includes a bending portion 4 that allows the base plate 3 to bend in a direction opposite to a surface of the base plate 3 on which the drug layer 2 is provided, and by bending the base plate 3 at the bending portion 4, at least a portion of a surface facing the base plate 3 of the drug layer 2 is exposed from the base plate 3.

## Description

### Technical Field

The present invention relates to support bodies having a drug that is to be administered to the anterior segment of the eye, and methods for producing the drug support body.

### Background Art

Eye drops are conventionally well known as ophthalmic drugs that are administered to the anterior segment of the eye. In eye drop administration, a liquid drug contained in an eye drop container is administered in drops. The amount of each drop may vary depending on pressure applied to the eye drop container, and may not be necessarily equal to the desired dose.

On the other hand, as a means for administering a predetermined amount of a drug to the anterior segment of the eye, a technique of using a solid composition as an ophthalmic drug and inserting the solid composition is known. For such an ophthalmic drug, a drug component(s) that needs to be administered at one time is contained in a single solid composition, and therefore, a predetermined amount of the drug can be administered. However, the drug is held directly by the hand, and therefore, it is not easy to maintain the drug in sterile condition.

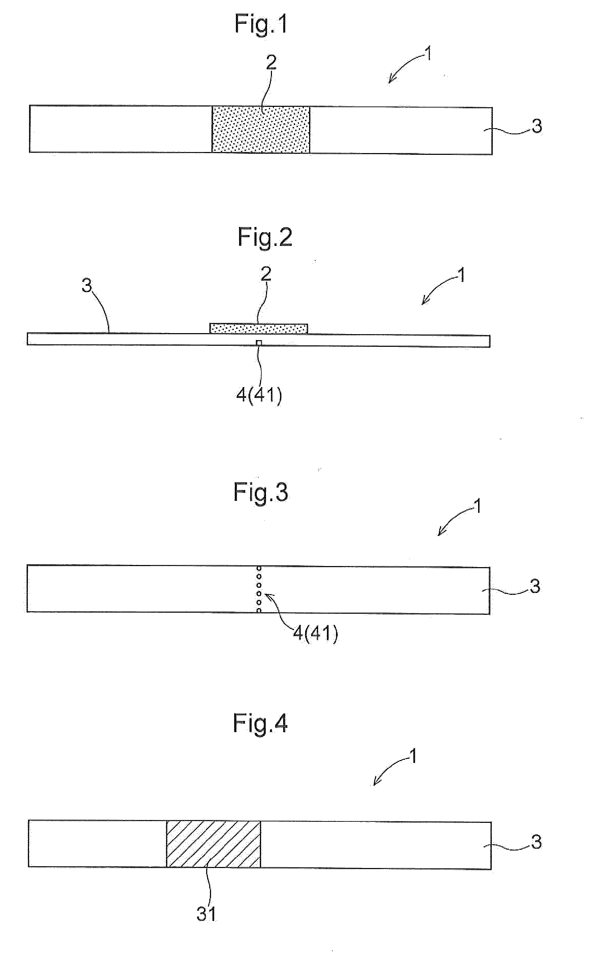

As an ophthalmic drug that overcomes the above problem, Patent Document 1 describes an applicator for administering a drug to a moist biological surface. Specifically, the applicator is a disposable applicator having an elongated strip shape at one end of which a drug containing a drug component in a soluble matrix is provided. The drug and the applicator are connected together via a separation portion formed of a soluble thin film (see the claims and figure 1). In the applicator, the drug itself is allowed to come into contact with the biological surface as an administration site while the other end portion opposite to the drug itself of the applicator is held. As a result, the separation portion is dissolved, so that the drug itself is separated from the applicator, and the drug itself is administered. Thus, the use of the disposable applicator facilitates maintenance of sterile condition during drug administration.

### Citation List

### Patent Document

Patent Document 1: JP H4-11229 B2

### Summary of Invention

### Technical Problem

However, in the above applicator, the applicator, the separation portion and the drug itself are disposed along the longitudinal direction. Typically, the drug itself and the separation portion, which are formed of a soluble matrix or the like, have a low strength. Therefore, in the applicator having the above configuration, the separation portion or the drug itself is likely to be damaged, and therefore, it is difficult to produce and handle the applicator.

The present invention has been made in view of the above background. It is an object of the present invention to provide a drug support body that can be used to appropriately administer a drug to the anterior segment of the eye while reducing the production cost.

### Solution to Problem

A drug support body according to the present invention includes a drug layer containing a drug component to be administered to an anterior segment of an eye, at a halfway portion in a longitudinal direction of a base plate, wherein the base plate includes a bending portion that allows the base plate to bend in a direction opposite to a surface of the base plate on which the drug layer is provided, and by bending the base plate at the bending portion, at least a portion of a surface facing the base plate of the drug layer is exposed from the base plate.

With this configuration, the drug layer is provided on the base plate, and therefore, the drug layer, whose strength is typically low, is protected by the base plate. Therefore, the drug layer can be prevented from being damaged, and therefore, it is easier to produce and handle the drug layer. When the base plate is bent at the bending portion, the surface facing the base plate of the drug layer is exposed from the base plate. Therefore, the base plate thus bent can be used as the above applicator. Therefore, the drug can be administered while being maintained in sterile condition.
The bending portion of the base plate is located at a tip end portion of the base plate serving as an applicator on the drug layer side. The bending portion typically has a round and smooth shape, and therefore, the tip end portion of the applicator can have a smooth shape without particularly processing the base plate. Therefore, when the drug layer is administered, the anterior segment of the eye can be prevented from being damaged.
In other words, in the case of the applicator described in Patent Document 1, the tip of the applicator needs to be smoothed in order to prevent the biological surface as an administration site from being damaged, resulting in the additional cost of processing the tip portion. As described above, the bending portion typically has a round and smooth shape, and therefore, the tip end portion of the applicator can have a smooth shape without particularly processing the base plate. Therefore, the cost of processing the applicator can be reduced.
As a result, a drug support body can be provided that can be used to appropriately administer a drug to the anterior segment of the eye while the production cost is reduced.

In the above embodiment, a portion facing the base plate of the drug layer may be exposed from the base plate in various forms. For example, the drug layer may be formed to extend over both sides of a portion of the base plate in which the bending portion is formed, and when the base plate is bent at the bending portion, a portion of the drug layer on one side of the bending portion may be maintained facing the base plate while another portion of the drug layer on the other side of the bending portion may be exposed from the base plate and protrudes from the bending portion. Alternatively, the drug layer may be formed to extend over both sides of a portion of the base plate in which the bending portion is formed, and when the base plate is bent at the bending portion, a predetermined portion of the drug layer including a portion corresponding to the bending portion may be maintained facing the base plate while portions on both sides of the predetermined portion may be exposed from the base plate.

A plurality of bending portions are preferably provided in the base plate, and the drug layer is preferably formed at a portion of the base plate in which one of the bending portions is formed.

With this configuration, when the base plate is bent at the bending portions, the base plate can be put into a generally polygonal shape, whereby the strength of the base plate after being bent can be increased. Also, the position or orientation of the drug layer can be changed by changing the polygonal shape, and therefore, the drug layer can be put into a position or orientation that allows the drug layer to be easily administered.

In the above configuration, of the portions of the drug layer sticking to the base plate, a portion that is released when the base plate is bent preferably has a sticking force smaller than that of the other portion.

With this configuration, when the base plate is bent, a portion of the drug layer having a lower sticking force is reliably released from the base plate due to the difference in the sticking force of the base plate and the drug layer. Therefore, the drug can be more accurately administered.

In the above configuration, a sticky layer is preferably provided between the drug layer and the base plate.

The drug layer may have a somewhat large sticking force. However, some drug layers may not have a sufficient sticking force depending on conditions such as types of drug layers. In this case, by providing a sticky layer between the drug layer and the base plate, the drug layer can be reliably formed on the base plate.

In the drug support body of the present invention, the present invention is not limited to the configuration in which the drug layer is provided on the base plate by causing the drug layer to stick to the base plate. For example, a holding member configured to hold the drug layer may be provided on the base plate, and the drug layer may be provided on the base plate by being sandwiched between the base plate and the holding member.

In the above configuration, a cover layer configured to cover the drug layer is preferably formed on an upper surface of the drug layer.

With this configuration, the drug layer can be reliably protected by the base plate and the cover layer, and therefore, it is easier to handle the drug layer.

In the above configuration, a fragile portion having a flexural strength lower than that of the other portion of the base plate is preferably formed in the bending portion.

With this configuration, when the base plate is bent, the base plate can be reliably bent at the fragile portion having a lower flexural strength than that of the other portion.

In the above configuration, a sign indicating a portion to be bent is preferably formed at the bending portion.

With this configuration, the user can visually recognize the bending portion, and therefore, can bend the base plate at the appropriate position.

A method for producing the drug support body of the present invention includes a drug layer forming step of forming the drug layer along a longitudinal direction of an original material for the base plate, a bending portion forming step of forming the bending portion along the longitudinal direction of the original material for the base plate, and a cutting step of, after the drug layer forming step and the bending portion forming step, cutting the original material of the base plate along a direction perpendicular to the longitudinal direction at a predetermined interval.

In a drug support body according to the present invention, a fragile portion having a strength lower than that of the other portion is formed at a halfway portion in a longitudinal direction of a base plate. The drug support body includes a drug layer containing a drug component to be administered to an anterior segment of an eye and formed on one surface of the base plate, extending across the fragile portion, a sandwiching body provided on the one surface of the base plate, extending from one end in the longitudinal direction of the drug layer, and configured so that the drug layer is sandwiched between the sandwiching body and the base plate, and a protection body extending from the other end in the longitudinal direction of the drug layer, over the upper surface of the drug layer and further at least a portion of an upper surface of the sandwiching body.

With this configuration, the drug layer is provided on the base plate. The drug layer, whose strength is typically low, is provided on the base plate. The drug layer is sandwiched between the base plate and the sandwiching body, and the protection body is provided on the upper surface of the drug layer. Therefore, the drug layer is protected and prevented from being damaged, and therefore, it is easier to produce and handle the drug layer.
The drug layer extends across the fragile portion that is formed at the halfway portion in the longitudinal direction of the base plate. Therefore, after the drug layer is exposed from the protection body, if the base plate is broken or bent along the fragile portion, the drug layer is exposed from the base plate, and therefore, the base plate can be used as the above applicator. Therefore, the drug can be administered while being maintained in sterile condition.
As described above, a drug support body can be provided that can be used to appropriately administer a drug to the anterior segment of the eye while the production cost is reduced.

In the above configuration, the base plate and a region extending from the one end to the halfway portion of the sandwiching body are preferably attached together, the drug layer is preferably sandwiched between the base plate and the sandwiching body in a portion in which the base plate and the sandwiching body are not attached together, and the protection body is preferably attached to a region extending from the other end of the base plate to at least a halfway portion in a longitudinal direction of the sandwiching body.

With this configuration, the drug layer can be reliably sandwiched in a portion in which the base plate and the sandwiching body are not attached together. Also, because the protection body is attached to a region extending from the other end of the base plate to at least a halfway portion in a longitudinal direction of the sandwiching body, the protection body is reliably located on the upper surface of the drug layer, whereby the drug layer can be reliably protected.

In the above configuration, the sandwiching body preferably extends over a region between an end portion at the one end of the base plate and the fragile portion, and the protection body preferably extends from an end portion at the other end of the base plate to the halfway portion of the sandwiching body.

With this configuration, the sandwiching body extends over a region between an end portion at the one end of the base plate and the fragile portion. Therefore, the sandwiching body is provided throughout a portion of the base plate that acts as an applicator. Therefore, the base plate can be reinforced by the sandwiching body. As a result, even when the base plate is formed of a material having an insufficient strength, the base plate can be reliably used as an applicator.

In the above configuration, a non-attached portion to which the sandwiching body is not attached is preferably formed in a predetermined region extending from an end portion of the protection body on the sandwiching body side.

With this configuration, when the drug layer is exposed from the protection body, the protection body can be released from the non-attached portion. As a result, it is easier to handle the drug support body when the drug is administered.

### Brief Description of Drawings

FIG. 1 is a top view showing an example drug support body according to a first embodiment of the present invention.
FIG. 2 is a side view showing an example drug support body according to a first embodiment of the present invention.
FIG. 3 is a bottom view showing an example drug support body according to a first embodiment of the present invention.
FIG. 4 is a top view showing an example base plate.
FIG. 5 is a diagram showing an example method of using a drug support body according to the present invention.
FIG. 6 is a diagram showing an example method for producing a drug support body according to the present invention.
FIG. 7 is a diagram showing a drug support body that has been hermetically sealed and packaged.
FIG. 8 is a top view showing an example base plate.
FIG. 9 is a side view showing an example drug support body according to a second embodiment of the present invention.
FIG. 10 is a side view showing an example drug support body according to a third embodiment of the present invention.
FIG. 11 is a diagram showing an example drug support body according to a fourth embodiment of the present invention.
FIG. 12 is a side view showing an example drug support body according to a fourth embodiment of the present invention.
FIG. 13 is a diagram showing an example method of using a drug support body according to the present invention.
FIG. 14 is a diagram showing a drug support body according to another embodiment.
FIG. 15 is a diagram showing a drug support body according to another embodiment.
FIG. 16 is a diagram showing a drug support body according to another embodiment.
FIG. 17 is a diagram showing a drug support body according to another embodiment.
FIG. 18 is a diagram showing a drug support body according to another embodiment.
FIG. 19 is a diagram showing a drug support body according to another embodiment.
FIG. 20 is a diagram showing a drug support body according to another embodiment.

### Description of Embodiments

Embodiments of a drug support body 1 according to the present invention will be described hereinafter with reference to the accompanying drawings. Note that the present invention is not limited to the embodiments below, and various changes and modification can be made without departing the scope and spirit of the present invention.

### [First Embodiment]

A first embodiment of the drug support body 1 of the present invention will be described with reference to the drawings.
As shown in FIGS. 1 to 3, the drug support body 1 includes a base plate 3, and a drug layer 2 provided on one surface of the base plate 3.

The base plate 3 has, for example, an elongated rectangular shape. The base plate 3 may be formed of any appropriate material, such as, but not particularly limited to, paper, plastic, aluminum foil or the like. The base plate 3 may also be formed of a stack of a plurality of layers of these materials.

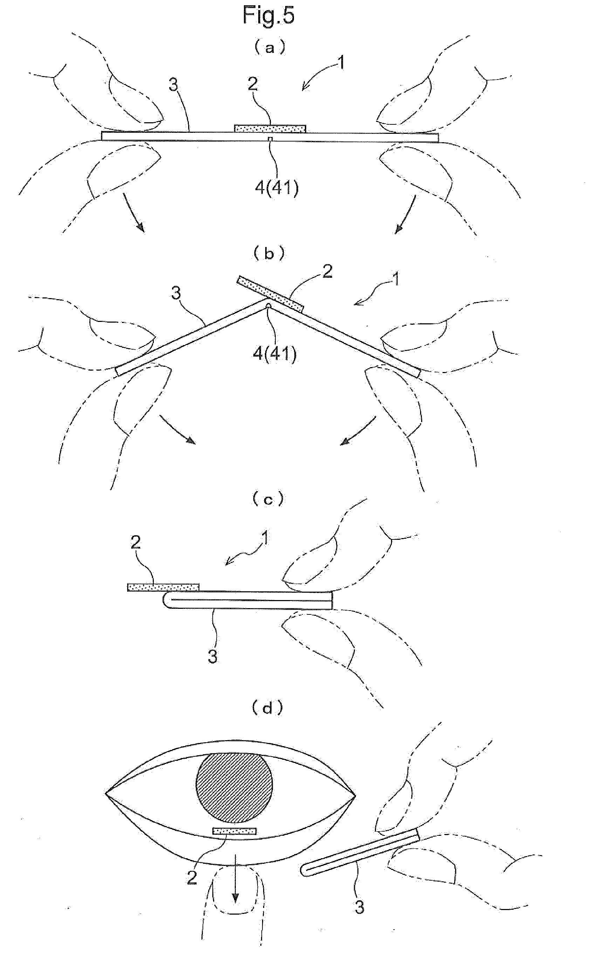

As shown in FIGS. 2 and 3, a bending portion 4 at which the base plate 3 can be bent is provided at a middle portion in a longitudinal direction of the base plate 3. In this embodiment, a perforation 41 is formed in the bending portion. The perforation 41 is provided in one surface of the base plate 3 that is opposite to another surface on which the drug layer 2 is provided, extending along a direction perpendicular to the longitudinal direction of the base plate 3. The holes of the perforation 41 do not completely pass through the base plate 3, and penetrate halfway through the base plate in the thickness direction. The perforation 41 thus formed in the bending portion 4 causes the bending portion 4 to have a lower flexural strength than that of the other portion of the base plate 3. As a result, when the base plate 3 is bent, the base plate 3 can reliably bend at the bending portion 4 (see FIG. 5(b)). The perforation 41 corresponds to a fragile portion of the present invention.

As shown in FIGS. 1 and 2, the drug layer 2 is formed on one surface of the base plate 3 that is opposite to another surface on which the perforation 41 is provided. The drug layer 2 is formed at a halfway portion in the longitudinal direction of the base plate 3. Specifically, the base plate 3 has regions that extend from both end portions toward the halfway portion in the longitudinal direction and in which the drug layer 2 is not formed, and a region that is located at the halfway portion and in which the drug layer 2 is formed. In this embodiment, the drug layer 2 is formed at a substantially middle portion in the longitudinal direction of the base plate 3, and the perforation 41 is located at a substantially middle portion in the longitudinal direction of the drug layer 2. The drug layer 2 is formed, for example, of a solid composition containing a drug component. As the solid composition, for example, a water-soluble polymer or the like may be employed. The solid composition of the drug layer 2 is dissolved after administration, so that the drug component is absorbed into the administration site. The drug layer 2 may include a plurality of layers, and the layers may have the same or different drug components. The drug layer 2 is administered to an anterior segment of the eye.

The drug layer 2 sticks to the base plate 3 by means of a sticking force of the solid composition. In this embodiment, the drug layer 2 (solid composition) is configured so that the drug layer 2 has different levels of sticking force with respect to the base plate on opposite sides of the bending portion 4. Specifically, for example, a surface of the base plate 3 on which the drug layer 2 is formed is formed of different materials so that one side of the bending portion has a higher releasability, resulting in a lower sticking force between the base plate 3 and the drug layer 2. In this embodiment, as shown in FIG. 4, a release film layer 31 having a higher releasability is formed in a predetermined region extending from the bending portion 4 to one end portion of the base plate 3 (i.e., a region extending to a portion somewhat closer to that end portion of the base plate than is the end portion of the drug layer). Therefore, when the base plate is bent, a portion of the drug layer that is attached to the portion having a high releasability of the base plate is released and protrudes from the bending portion (see FIGS. 5(b) and 5(c)). Thus, at least a portion of a surface facing the base plate 3 of the drug layer 2 is exposed from the base plate 3. Note that the release film layer 31 may be formed throughout the region extending from the bending portion 4 to the one end portion of the base plate 3.

Next, a method of using the drug support body 1 will be described. Although an example case where the drug layer 2 is administered to the surface of the eye will be described, the drug layer 2 may be administered to an anterior segment of the eye, such as, for example, cornea, palpebral conjunctiva, conjunctival sac or the like. As shown in FIGS. 5(a) and 5(b), the user holds the base plate 3 at portions in the vicinity of both end portions thereof using fingers, and bends the base plate 3 in a direction opposite to the surface on which the drug layer 2 is formed. In other words, the base plate 3 is bent so that surfaces opposite to the drug layer 2, of the portions in the vicinity of both end portions of the base plate 3, face each other. As a result, as shown in FIG. 5(c), the base plate 3 is bent at a portion thereof corresponding to the perforation 41 formed in the bending portion 4, and the surfaces opposite to the drug layer 2 abut each other, i.e., the base plate 3 is doubled up. In this case, as described above, a portion of the drug layer 2 is exposed from the base plate 3 and protrudes from the bending portion.

As shown in FIG. 5(c), the user holds, using fingers, the folded base plate 3 at a portion in the vicinity of an end portion thereof opposite to the drug layer 2, i.e., the portions in the vicinity of both end portions of the base plate 3 before being folded, and moves the drug layer 2 toward the administration site. In this embodiment, as shown in FIG. 5(d), the user pulls down the lower eyelid using a finger of one hand to expose the conjunctiva, and moves the drug layer 2 toward the eye surface while holding the base plate 3 at the portions in the vicinity of the end portion opposite to the drug layer 2 using fingers of the other hand. The user causes a portion of the drug layer 2 protruding from the base plate 3 to come into contact with the eye surface so that the drug layer 2 is completely released from the base plate 3, whereby the drug layer 2 is administered to the eye surface. As described above, the folded base plate 3 functions as a support body that delivers the drug layer 2 to the administration site during administration of the drug layer 2. With the above configuration, when the base plate 3 functions as a support body, the base plate 3 is doubled up, and the presence of the folded portion increases the strength of the base plate 3. Therefore, when the base plate 3 functions as a support body, the base plate 3 is prevented from being accidentally deformed, whereby the drug layer 2 can be accurately delivered to the administration site.
Note that, in the above embodiment, a separation portion (e.g., a perforation etc.) may be provided in the drug layer 2 itself (e.g., a drug film etc.) at or close to a position corresponding to the perforation 41 of the base plate 3. In this embodiment, when the user folds the base plate (see FIG. 5(c)), a portion of the drug layer 2 is exposed from the base plate 3 and protrudes from the bending portion, and the separation portion (e.g., a perforation etc.) is located at or close to the bending portion of the drug layer 2. Thereafter, when the user causes the drug layer 2 to come into contact with the administration site (e.g., the eye surface etc.) in order to administer the drug layer 2 to the administration site (see FIG. 5(d)), the drug layer 2 is separated at the separation portion, so that a portion of the drug layer 2 protruding from the bending portion is administered to the administration site. On the other hand, the other portion of the drug layer 2 is left on the base plate 3. Thus, also in the embodiment in which the separation portion is provided in the drug layer 2, the drug layer 2 can be accurately administered to the administration site.

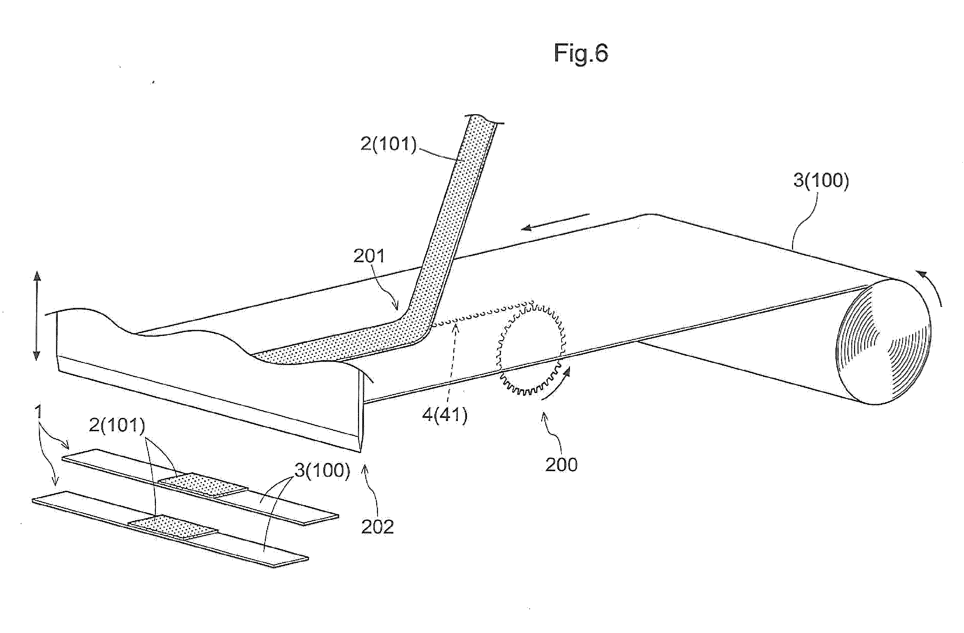

Next, an example method for producing the drug support body 1 will be described.
As shown in FIG. 6, an original material 100 for the base plate 3 is, for example, a roll of a long strip-shaped material. The original material 100 is supplied, sequentially from the leading end thereof, to a bending portion formation unit 200. In the bending portion formation unit 200, the perforation 41 is formed in one surface of the original material 100 that is opposite to another surface on which the drug layer 2 is to be formed. The perforation 41 is continuously formed in a middle portion in a width direction of the original material 100 along a longitudinal direction (a direction in which the original material is transported). The holes of the perforation 41 do not completely pass through the original material 100, and penetrate halfway through the original material 100 in the thickness direction. The bending portion formation unit 200 includes a member that can continuously form holes of the perforation 41 in the case of forming the perforation 41 in the bending portion 4, such as, for example, but not limited to, a needle member, a perforation cutter or the like.

The original material 100 in which the perforation 41 has been formed is transported to a drug layer formation unit 201 that is provided downstream in the transport direction. In the drug layer formation unit 201, the drug layer 2 is formed on one surface of the original material 100 that is opposite to another surface in which the perforation 41 has been formed. The drug layer 2 is formed at a halfway portion in the width direction of the original material. As the drug layer 2, for example, a long strip-shaped drug film 101 including a solid composition containing a drug component is employed. Note that, as the drug layer 2, a solid composition containing a drug component may be applied to the original material 100 instead of the drug film 101. As shown in FIG. 6, as the original material 100 is transported, the drug film 101 is caused to stick to the halfway portion in the width direction of the original material 100, so that the drug layer 2 is continuously formed along the longitudinal direction (i.e., the transport direction) of the original material 100. Alternatively, a solution in which a drug component is dissolved may be cast to the original material 100 to form the drug film 101 as the drug layer 2.

The original material 100 on which the drug layer 2 has been formed is transported to a cutting unit 202 that is provided downstream of the drug layer formation unit 201. In the cutting unit 202, the original material 100 is cut along a direction perpendicular to the longitudinal direction at a predetermined interval. The cutting unit 202 includes, for example, but not limited to, a cutter or the like that can cut the original material 100. As a result, the drug support body 1 whose longitudinal direction is the width direction of the original material 100 is formed.

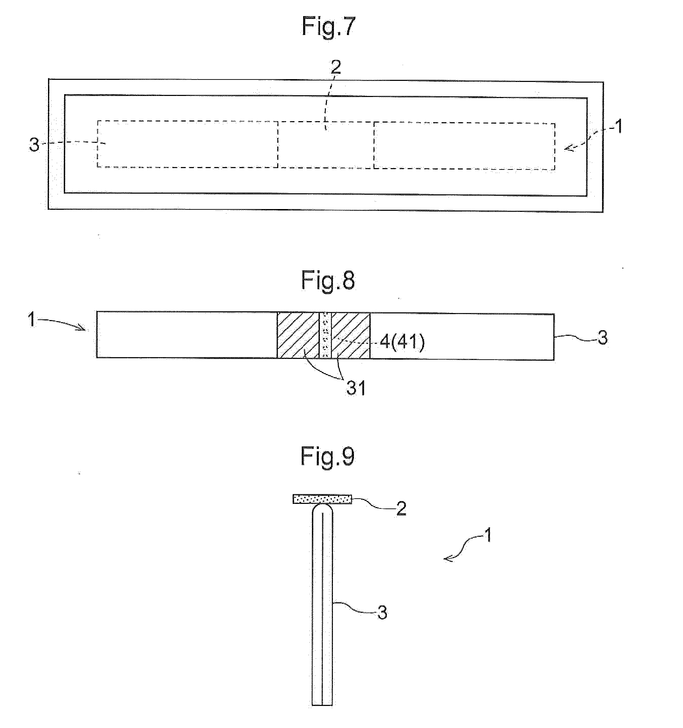

Thereafter, as shown in FIG. 7, the drug support body 1 is individually hermetically sealed and packaged. The drug support body 1 in the hermetically sealed package is sterilized, for example, by irradiation with ultraviolet light, y rays, electron beams or the like. As a result, the drug support body 1 is hermetically sealed and packaged in sterile condition.

### [Second Embodiment]

A second embodiment of the drug support body 1 of the present invention will be described with reference to the drawings.
In this embodiment, the sticking force of the drug layer 2 (solid composition) to the base plate 3 varies between a predetermined region including the bending portion 4 and the other region. Specifically, for example, as shown in FIG. 8, in the longitudinal direction of the base plate 3, the surface of the base plate 3 on which the drug layer 2 is formed is formed of different materials in different regions (i.e., the bending portion 4 and predetermined regions on both sides of the bending portion 4 (at-and-near-bending-portion region), and regions closer to the end portions than is the at-and-near-bending-portion region) so that the regions closer to the end portions than is the at-and-near-bending-portion region have a higher releasability and therefore a smaller sticking force between the base plate 3 and the drug layer 2. Specifically, the release film layer 31 having a higher releasability is formed in opposite predetermined regions (regions extending to portions somewhat closer to the end portions of the base plate than are the end portions of the drug layer) excluding a predetermined region in the vicinity of the bending portion 4. Therefore, when the base plate 3 is bent, a portion of the drug layer 2 that is attached to the portion (the release film layer 31) having a higher releasability of the base plate is released. Therefore, as shown in FIG. 9, the regions closer to both end portions of the drug layer 2 are released and exposed from the base plate 3 while only a portion corresponding to the at-and-near-bending-portion region of the drug layer 2 (i.e., a portion of the drug layer 2 that is located in the vicinity of a middle portion in the longitudinal direction) is attached to the base plate 3. Note that the release film layer 31 may extend to the end portion of the base plate 3.

As described above, in the drug support body 1 of the second embodiment, the drug layer 2 protrudes from the base plate 3, excluding the portion in the vicinity of the middle portion in the longitudinal direction. Therefore, when the drug layer 2 is administered, the base plate 3 can be prevented from coming into contact with the administration site, i.e., the anterior segment of the eye. Moreover, after the drug layer 2 comes into contact with the anterior segment of the eye, the drug layer 2 can be easily released from the base plate 3. Because the drug layer 2 protruding substantially perpendicularly to the base plate 3 comes into contact with the anterior segment of the eye, the drug support body 1 is moved toward the anterior segment of the eye from the front. Therefore, for example, when the drug layer 2 is administered to the anterior segment of an eye opposite to a hand holding the drug support body 1, it is less difficult for the nose to interfere with the drug support body 1 than when the drug support body 1 is moved toward the anterior segment of the eye from a diagonal direction. As a result, the drug layer 2 can be more easily administered.

### [Third Embodiment]

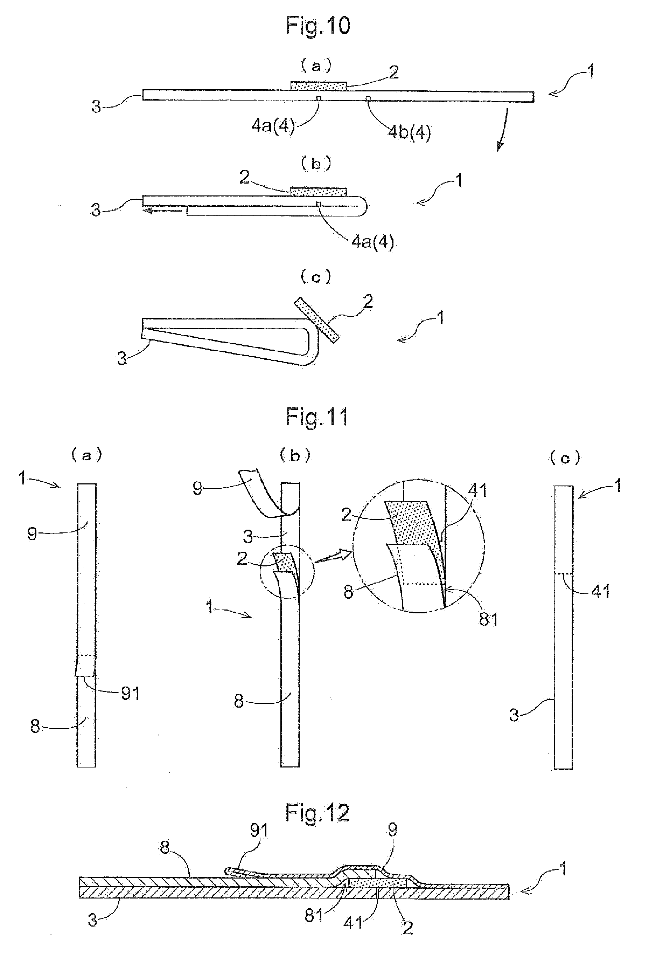

A third embodiment of the drug support body 1 of the present invention will be described with reference to the drawings.
In an example shown in FIG. 10, bending portions 4a and 4b are formed at two points of the base plate. The bending portion 4a is located at a substantially middle portion in the longitudinal direction of the drug layer 2. The bending portion 4b above which the drug layer 2 is not formed is located at a point that is closer to one end portion of the base plate 3 than is the middle portion in the longitudinal direction of the base plate 3. The bending portion 4a above which the drug layer 2 is formed is formed in a region whose distance between the bending portion 4b and the end portion of the base plate 3 is longer. In this embodiment, as in the second embodiment, the surface of the base plate on which the drug layer 2 is formed is formed of different materials in different regions (i.e., the bending portion 4a and predetermined regions on both sides of the bending portion 4a (at-and-near-bending-portion region), and regions closer to the end portions than is the at-and-near-bending-portion region) so that the regions closer to the end portions than is the at-and-near-bending-portion region have a higher releasability and therefore a smaller sticking force between the base plate 3 and the drug layer 2. As in the second embodiment, when the base plate is bent, the regions closer to both end portions of the drug layer 2 are released and exposed from the base plate 3 while only a portion corresponding to the at-and-near-bending-portion region of the drug layer 2 (i.e., a portion of the drug layer 2 that is located in the vicinity of a middle portion in the longitudinal direction) is attached to the base plate 3 (see FIG. 10(c)).

As described above, in the drug support body 1 of the third embodiment, the drug layer 2 protrudes from the base plate 3, excluding the portion in the vicinity of the middle portion in the longitudinal direction. Therefore, when the drug layer 2 is administered, the base plate 3 can be prevented from coming into contact with the administration site, i.e., the anterior segment of the eye. Moreover, after the drug layer 2 comes into contact with the anterior segment of the eye, the drug layer 2 can be easily released from the base plate 3. As a result, the drug layer 2 can be more easily administered.

Note that, as in the first embodiment, the surface of the base plate on which the drug layer 2 is formed may be formed of different materials on opposite sides of the bending portion 4a. In this case, as in the first embodiment, when the base plate is bent, a portion of the drug layer 2 that is attached to the portion having a higher releasability of the base plate 3 is released and protrudes from the bending portion.

Next, a method of using the drug support body 1 will be described. As shown in FIG. 10(a), the base plate 3 is bent at the bending portion 4b above which the drug layer 2 is not formed. In this case, as shown in FIG. 10(b), an end portion of the base plate 3 on one side of the bending portion 4b on which the drug layer 2 and the bending portion 4a are formed protrudes from an end portion of the base plate 3 on the other side. As shown in FIG. 10(b), portions in the vicinity of both end portions of the base plate 3 are relatively moved in directions that allow both end portions of the base plate 3 to move toward each other, whereby the base plate 3 is bent at the bending portion 4a in a direction opposite to the surface on which the drug layer 2 is formed. As a result, as shown in FIG. 10(c), the base plate 3 is bent into a generally triangular shape, and the drug layer 2 is located at a vertex of the triangle. Only the portion in the vicinity of the middle portion of the drug layer 2 is attached to the base plate 3 while both end portions thereof are released and exposed from the base plate 3. The user holds, using fingers, the base plate 3 at portions in the vicinity of both end portions of the base plate 3 as they were before the base plate 3 was folded, and administers the drug layer 2 to the administration site. In this case, by relatively moving the end portions of the base plate 3 to change the triangular shape, the drug layer 2 can be easily put into a position or orientation that allows the drug layer 2 to be easily administered to the administration site. The generally triangular shape of the base plate 3 increases the strength of the base plate 3. Therefore, when the base plate 3 functions as a support body, the base plate 3 can be prevented from being accidentally deformed, whereby the drug layer 2 can be accurately delivered to the administration site.

### [Fourth Embodiment]

A fourth embodiment of the drug support body 1 of the present invention will be described with reference to the drawings.
As shown in FIGS. 11 and 12, this drug support body 1 includes a base plate 3, a drug layer 2 that is provided on the base plate 3, a sandwiching body 8 that is provided so that a portion of the drug layer 2 is sandwiched between the sandwiching body 8 and the base plate 3, and a protection body 9 that protects the drug layer 2 on the base plate 3.

The base plate 3 has, for example, an elongated rectangular shape. The base plate 3 may be formed of any appropriate material, such as, but not particularly limited to, paper, plastic, aluminum foil or the like. The base plate 3 may also be formed of a stack of a plurality of layers of these materials. A surface treatment or the like may be performed as required on the base plate 3, taking into consideration, for example, the releasability of the drug layer 2 from the base plate 3 when the drug is administered.

The protection body 9 has, for example, but not particularly limited to, an elongated rectangular shape. The protection body 9 is formed of, for example, but not particularly limited to, resin film, particularly preferably transparent film. The sandwiching body 8 also has, for example, but not particularly limited to, an elongated rectangular shape. The sandwiching body 8 may be formed of, for example, but not particularly limited to, aluminum foil.
Note that the materials for the protection body 9 and the sandwiching body 8 are not limited to the above materials, and aluminum-deposited film, alumina-deposited transparent film, silica-deposited transparent film, paper, plastic, aluminum foil or the like may be employed as appropriate.

As shown in FIGS. 11 and 12, the base plate 3 has a fragile portion at a halfway portion in the longitudinal direction. The fragile portion has a lower strength than that of the other portion so that the base plate can be bent or broken at the fragile portion. In this embodiment, a perforation 41 is formed in the fragile portion. In this embodiment, the perforation 41 passes through the base plate 3. The fragile portion is thus configured by forming the perforation 41 so that the strength is weaker than that of the other portion of the base plate 3. As a result, the base plate 3 can be reliably bent or broken at the fragile portion. Note that the perforation 41 may not pass through the base plate 3 in the thickness direction, and may penetrate halfway through the base plate 3 in the thickness direction. The fragile portion is not limited to the perforation 41, and may be, for example, a portion having a cut penetrating halfway in the thickness direction, a portion having a smaller thickness than that of the other portion, a portion having a crease previously formed on the base plate and therefore a lower strength than that of the other portion, or the like, instead of the perforation.

As shown in FIG. 12, the drug layer 2 is formed on one surface of the base plate 3. The drug layer 2 extends across the fragile portion (the halfway portion in the longitudinal direction of the drug layer 2 is located above the fragile portion). In other words, in the longitudinal direction of the base plate 3, the base plate 3 has regions that extend from both end portions toward the halfway portion and in which the drug layer 2 is not formed, and a region that includes the perforation 41 of the halfway portion and in which the drug layer 2 is formed. The drug layer 2 is formed of, for example, a solid composition containing a drug component. As the solid composition, for example, a water-soluble polymer or the like may be employed. The solid composition of the drug layer 2 is dissolved after administration, so that the drug component is absorbed into the administration site. The drug layer 2 may include a plurality of layers, and the layers may have the same or different drug components. The drug layer 2 is administered to an anterior segment of the eye.

In this embodiment, the sandwiching body 8 is provided on the surface of the base plate 3 on which the drug layer 2 is provided, extending from one end in the longitudinal direction of the surface to the portion in which the perforation 41 is formed. As a result, a portion of the drug layer 2 is sandwiched between the sandwiching body 8 and the base plate 3. In this embodiment, a heat seal layer is provided on the surface of the base plate 3 on which the drug layer 2 is provided. The base plate and the sandwiching body 8 are integrated together by heat sealing. Here, a portion of the sandwiching body 8 that is in the vicinity of the end portion closer to the perforation 41 is not attached to the base plate 3 by heat sealing, and forms a sandwiching space 81 in which the drug layer 2 is sandwiched (see FIGS. 11 and 12). Although, in this embodiment, the perforation 41 and the end portion of the sandwiching body 8 coincide in position, the perforation 41 and the end portion of the sandwiching body 8 do not necessarily coincide in position. Alternatively, for example, the end portion of the sandwiching body 8 may be located slightly short of the perforation 41 or slightly beyond the perforation 41.

In this embodiment, the protection body 9 is provided on the surface of the base plate 3 on which the drug layer 2 is provided, extending from the other end (the end opposite to the side on which the sandwiching body 8 is provided) to a halfway portion in the longitudinal direction of the sandwiching body 8. As a result, the protection body 9 is provided over the drug layer 2. As described above, the heat seal layer is provided on the base plate 3 to attach the protection body 9 and the base plate 3 together by heat sealing. The sandwiching body 8 and the protection body 9 are also attached together by heat sealing.

In this embodiment, the protection body 9 has a non-attached portion 91 to which the sandwiching body 8 is not attached, at an end portion thereof on the sandwiching body 8 side. Specifically, a part of the end portion of the protection body 9 on the sandwiching body 8 side is folded in two parts, and the two parts are fused together by melting, whereby the resulting part has a higher strength than that of the other portion and is prevented from being attached to the sandwiching body 8. Note that the non-attached portion 91 is not limited to the above form, and alternatively, for example, the end portion of the protection body 9 on the sandwiching body 8 side may have any form that prevents the end portion from being attached to the sandwiching body 8 without folding the end portion in two parts and fusing the two parts by melting. The non-attached portion 91 acts as a tab to remove the protection body 9 during use of the drug support body 1.
Note that the non-attached portion 91 may not be necessarily provided.

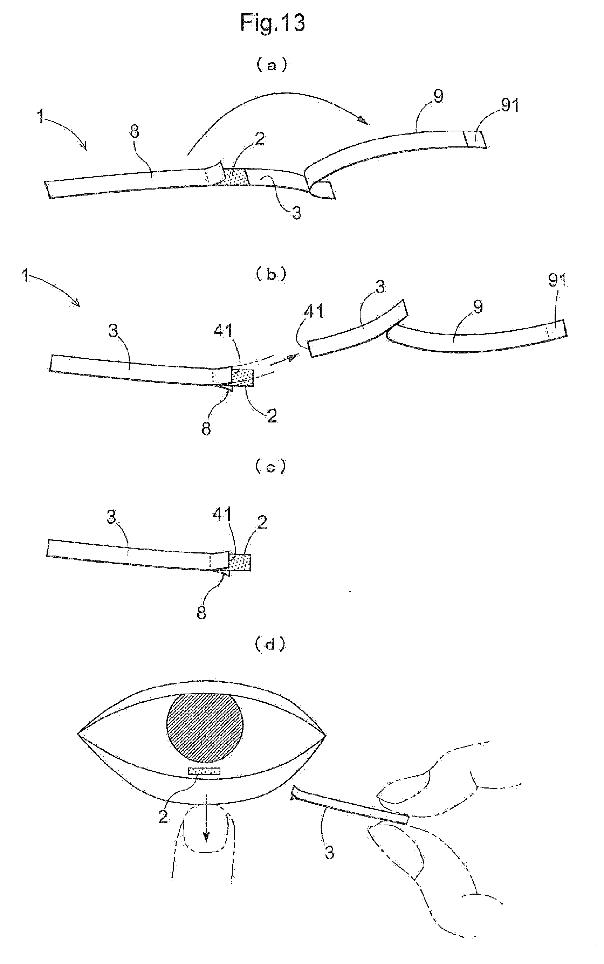

Next, a method of using the drug support body 1 will be described with reference to FIG. 13. Although an example case where the drug layer 2 is administered to the surface of the eye will be described, the drug layer 2 may be administered to an anterior segment of the eye, such as, for example, cornea, palpebral conjunctiva, conjunctival sac or the like. As shown in FIG. 13(a), the user removes the protection body 9 from the sandwiching body 8 by holding the tab (the non-attached portion 91) formed on the protection portion 9. In this case, the protection body 9 is not necessarily completely released from the base plate 3, and only needs to be released from the base plate 3 beyond the perforation 41. Thereafter, as shown in FIG. 13(b), the user breaks the base plate 3 along the perforation 41 to remove the protection portion 9 and one part of the base plate 3 on which the sandwiching body 8 is not provided. As a result, a portion of the drug layer 2 is sandwiched in the sandwiching space 81 between the base plate 3 and the sandwiching body 8, while the other portion of the drug layer 2 is exposed from the base plate 3 and the sandwiching body 8 (see FIG. 13(c)).

As shown in FIG. 13(c), the user moves the drug layer 2 toward the administration site while holding a portion of the base plate 3 and the sandwiching body 8 that is located in the vicinity of an end portion opposite to the drug layer 2. In this embodiment, as shown in FIG. 13(d), the user pulls down the lower eyelid using a finger of one hand to expose the conjunctiva, and moves the drug layer 2 toward the eye surface while holding the portion in the vicinity of the end portion of the base plate 3 and the sandwiching body 8 using fingers of the other hand. The user causes a portion of the drug layer 2 protruding from the base plate 3 to come into contact with the eye surface so that the drug layer 2 is completely released from the base plate 3, whereby the drug layer 2 is administered to the eye surface. As described above, the base plate 3 and the sandwiching body 8 function as a support body that delivers the drug layer 2 to the administration site during administration of the drug layer 2.

### [Other Embodiments]

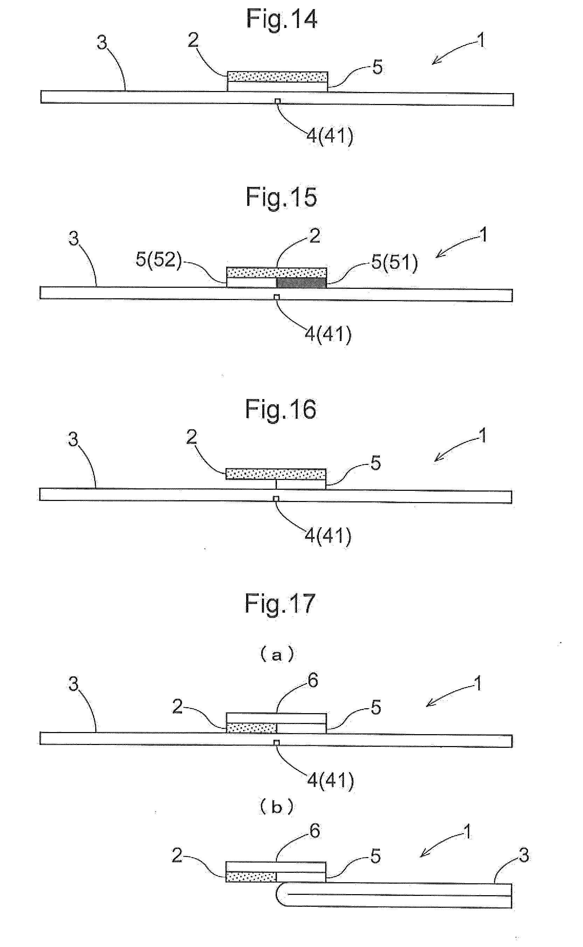

(1) In the above embodiments, the drug layer 2 is provided directly on the base plate 3 as an example. Alternatively, a sticky layer 5 may be provided between the base plate 3 and the drug layer 2.
   In this case, as shown in FIG. 14, a single sticky layer 5 may be provided throughout the drug layer 2.
   Alternatively, as shown in FIG. 15, different sticky layers 5 may be provided on opposite sides of the bending portion 4 (the perforation 41), i.e., a highly sticky layer 51 having a high sticking force and a less sticky layer 52 having a smaller sticking force than that of the highly sticky layer 51, may be formed. Note that when this embodiment is applied to an embodiment similar to the second or third embodiment, the highly sticky layer 51 may be formed on a portion in the vicinity of the bending portion 4 (4a), and the less sticky layer 52 may be formed on opposite sides of that portion.
   As shown in FIG. 16, the sticky layer 5 may be provided on only one side of the bending portion 4 (the perforation 41). Note that when this embodiment is applied to an embodiment similar to the second or third embodiment, the sticky layer 5 may be provided on only a portion in the vicinity of the bending portion 4 (4a).

(2) In the above embodiments, different portions of the surface of the base plate 3 are formed of different materials so that the different portions have different sticking forces. Alternatively, instead of providing different materials, the different portions may be caused to have different sticking forces by changing the surface condition (e.g., surface roughness etc.) of the base plate. The different portions may not necessarily need to have different sticking forces.

(3) In the above embodiments, the perforation 41 is formed as the fragile portion as an example. The present invention is not limited to this. Alternatively, the fragile portion may be any configuration that has a lower flexural strength than that of the other portion of the base plate 3, including, for example, a portion having a cut penetrating halfway in the thickness direction, a groove portion, a portion having a smaller thickness than that of the other portion, or the like. Although, as described above, the fragile portion is preferably provided in one surface of the base plate 3 that is opposite to another surface on which the drug layer 2 is provided, the present invention is not limited to this. The fragile portion may be provided on the surface of the base plate 3 on which the drug layer 2 is provided. Alternatively, the fragile portion may be provided on both surfaces of the base plate 3.

(4) The fragile portion may not necessarily need to be provided. Note that when the fragile portion is not provided, a sign indicating a bending portion is preferably provided on the base plate so that the user can find the bending portion. Examples of the sign include, but are not particularly limited to, a dashed line, a solid line and the like. Note that both the fragile portion and the sign may be provided.

(5) As shown in FIG. 17, a cover layer 6 may be provided on an upper surface of the drug layer 2. The material for the cover layer 6 may be, for example, but not limited to, biodegradable polymer, water-soluble polymer or the like as appropriate. Note that the cover layer may have an ability to stick to the administration site in order to allow the drug layer 2 to be accurately administered to the administration site. In the example of FIG. 17, the drug layer 2 is provided on one side of the bending portion 4 and the sticky layer 5 is provided on the other side, and the cover layer 6 is provided, extending over the upper surfaces of the drug layer 2 and the sticky layer 5.

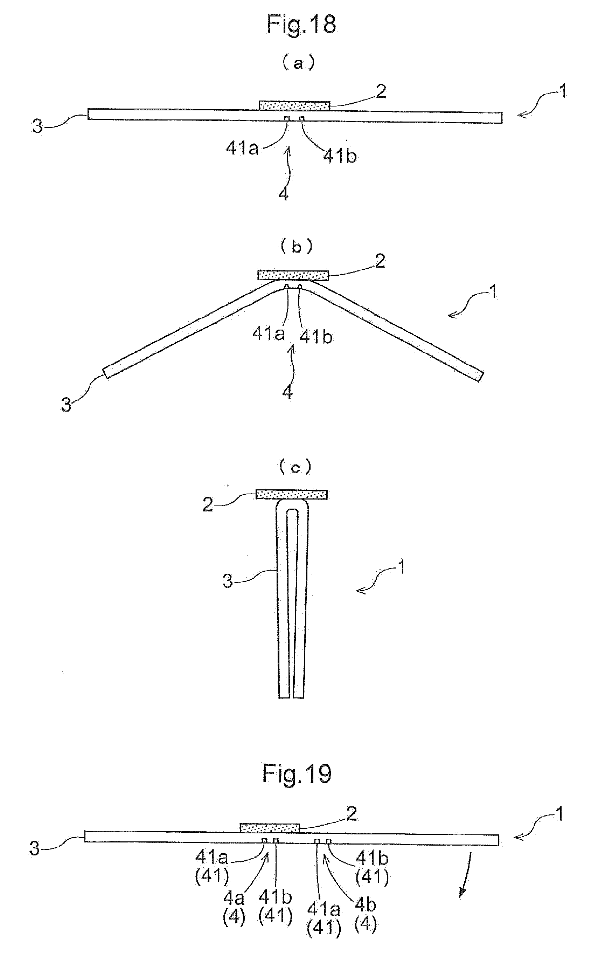

(6) In the above embodiments, the bending portion 4 is a line of the perforation 41. Alternatively, the bending portion 4 may be a plurality of adjacent lines of perforations 41. For example, in the case of the second embodiment, as shown in FIG. 18(a), the bending portion 4 is two adjacent lines of perforations 41a and 41b. Note that the bending portion 4 may be three or more lines of perforations 41. In this embodiment, as in the above embodiments, when the user bends the base plate 3 in a direction opposite to the surface on which the drug layer 2 is formed while holding portions in the vicinity of both end portions of the base plate 3 using fingers, as shown in FIG. 18(b) the base plate 3 is bent at a portion in which the perforations 41a and 41b are formed. As a result, as shown in FIG. 18(c), the drug layer 2 is released and exposed from the base plate 3, excluding a portion thereof which is formed in a region of the base plate 3 between the two perforations 41a and 41b. In other words, outer regions in the longitudinal direction of the drug layer 2 are released and exposed from the base plate 3 while a region in the vicinity of the middle portion in the longitudinal direction of the drug layer 2 is attached to the region of the base plate 3 between the perforations 41a and 41b.

Even in the other embodiments, the bending portion 4 may be a plurality of adjacent lines of perforations 41. FIG. 19 shows an example of the third embodiment in which the bending portions 4a and 4b are each a plurality of adjacent lines of perforations 41. In this embodiment, the bending portions 4a and 4b are each two adjacent lines of perforations 41a and 41b. Note that, even in any of the other embodiments, the bending portion 4 may be similarly a plurality of adjacent lines of perforations 41.

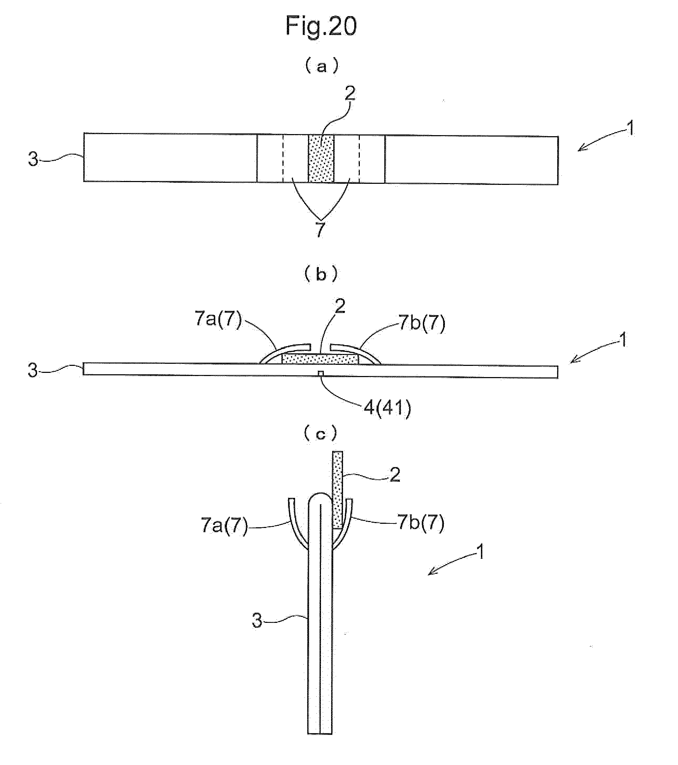

(7) In the above embodiments, when the drug layer 2 is formed on the base plate 3, the drug layer 2 sticks to the base plate 3 as an example. The present invention is not limited to this. For example, the drug layer 2 may be physically held on the base plate 3. In this embodiment, two holding members 7 (7a, 7b) are provided in the vicinity of a middle portion of the base plate. As shown in FIGS. 20(a) and 20(b), outer end portions (fixed ends) of the holding members 7a and 7b in the longitudinal direction of the base plate 3 are fixed to the base plate 3, while inner end portions (free ends) of the holding members 7a and 7b in the longitudinal direction of the base plate 3 are not fixed to the base plate 3. The drug layer 2 is held on the base plate 3 by being sandwiched between the holding members 7a and 7b and the base plate 3. Thus, the drug layer 2 is provided on the base plate 3. Although, in the above example, the two holding members 7a and 7b are provided as an example, there may be one or three or more holding members 7.

The holding member 7 (7a, 7b) is not particularly limited, and is preferably formed of a flexible material, such as, for example, plastic, paper, film or the like. The holding member 7 (7a, 7b) is preferably formed of a transparent or translucent material to allow the user to see the drug layer 2 through the holding member 7. When the holding members 7a and 7b are bent, the drug layer 2 is inserted between the base plate 3 and the holding members 7a and 7b.

When, as in the above embodiments, the user bends the base plate 3 in a direction opposite to the surface on which the drug layer 2 is formed while holding portions in the vicinity of both end portions of the base plate 3 using fingers, one (7a) of the holding members 7a and 7b is bent, so that the drug layer 2 is no longer sandwiched between the holding member 7a and the base plate 3. As a result, as shown in FIG. 20(c), only one end portion of the drug layer 2 is sandwiched between the base plate 3 and the holding member 7b, and the other end of the drug layer 2 (i.e., the portion that was sandwiched between the base plate 3 and the holding member 7a) protrudes from the base plate 3. Thus, a portion of the drug layer 2 is exposed from the base plate 3. As in the above embodiments, the user can deliver the drug layer 2 to the administration site using the folded base plate 3 as a support body.

### Industrial Applicability

The present invention is applicable to support bodies having a drug that is to be administered to the anterior segment of the eye, and methods for producing the support body.

### Reference Signs List

| | |
|---|---|
| 1 | DRUG SUPPORT BODY |
| 2 | DRUG LAYER |
| 3 | BASE PLATE |
| 31 | RELEASE FILM LAYER |
| 4 | BENDING PORTION (FRAGILE PORTION) |
| 41 | PERFORATION OR HOLES OF PERFORATION |
| 5 | STICKY LAYER |
| 51 | HIGHLY STICKY LAYER |
| 52 | LESS STICKY LAYER |
| 6 | COVER LAYER |
| 7 | HOLDING MEMBER |
| 8 | SANDWICHING BODY |
| 81 | SANDWICHING SPACE |
| 9 | PROTECTION BODY |
| 91 | NON-ATTACHED PORTION |
| 100 | ORIGINAL MATERIAL |
| 101 | DRUG FILM |
| 200 | BENDING PORTION FORMATION PORTION |
| 201 | DRUG LAYER FORMATION PORTION |
| 202 | CUTTING UNIT |

## Claims

1. A drug support body comprising :
a drug layer containing a drug component to be administered to an anterior segment of an eye, at a halfway portion in a longitudinal direction of a base plate,
wherein the base plate includes a bending portion that allows the base plate to bend in a direction opposite to a surface of the base plate on which the drug layer is provided, and by bending the base plate at the bending portion, at least a portion of a surface facing the base plate of the drug layer is exposed from the base plate.

2. The drug support body according to claim 1,
wherein the drug layer is formed to extend over both sides of a portion of the base plate in which the bending portion is formed, and when the base plate is bent at the bending portion, a portion of the drug layer on one side of the bending portion is maintained facing the base plate while another portion of the drug layer on the other side of the bending portion is exposed from the base plate and protrudes from the bending portion.

3. The drug support body according to claim 1,
wherein the drug layer is formed to extend over both sides of a portion of the base plate in which the bending portion is formed, and when the base plate is bent at the bending portion, a predetermined portion of the drug layer including a portion corresponding to the bending portion is maintained facing the base plate while portions on both sides of the predetermined portion are exposed from the base plate.

4. The drug support body according to any one of claims 1 to 3,
wherein a plurality of bending portions are provided in the base plate, and the drug layer is formed at a portion of the base plate in which one of the bending portions is formed.

5. The drug support body according to any one of claims 1 to 4,
wherein, of the portions of the drug layer sticking to the base plate, a portion that is released when the base plate is bent has a sticking force smaller than that of the other portion.

6. The drug support body according to any one of claims 1 to 5,
wherein a sticky layer is provided between the drug layer and the base plate.

7. The drug support body according to any one of claims 1 to 6,
wherein a holding member configured to hold the drug layer is provided on the base plate, and the drug layer is provided on the base plate by being sandwiched between the base plate and the holding member.

8. The drug support body according to any one of claims 1 to 7,
wherein a cover layer configured to cover the drug layer is formed on an upper surface of the drug layer.

9. The drug support body according to any one of claims 1 to 8,
wherein a fragile portion having a flexural strength lower than that of the other portion of the base plate is formed in the bending portion.

10. The drug support body according to any one of claims 1 to 9,
wherein a sign indicating a portion to be bent is formed at the bending portion.

11. A method for producing the drug support body according to any one of claims 1 to 10, comprising:
a drug layer forming step of forming the drug layer along a longitudinal direction of an original material for the base plate;
a bending portion forming step of forming the bending portion along the longitudinal direction of the original material for the base plate; and
a cutting step of, after the drug layer forming step and the bending portion forming step, cutting the original material of the base plate along a direction perpendicular to the longitudinal direction at a predetermined interval.

12. A drug support body comprising :
wherein a fragile portion having a strength lower than that of the other portion is formed at a halfway portion in a longitudinal direction of a base plate, and
a drug layer containing a drug component to be administered to an anterior segment of an eye and formed on one surface of the base plate, extending across the fragile portion, and
a sandwiching body provided on the one surface of the base plate, extending from one end in the longitudinal direction of the drug layer, and configured so that the drug layer is sandwiched between the sandwiching body and the base plate, and
a protection body extending from the other end in the longitudinal direction of the drug layer, over the upper surface of the drug layer and further at least a portion of an upper surface of the sandwiching body.

13. The drug support body according to claim 12,
wherein the base plate and a region extending from the one end to the halfway portion of the sandwiching body are attached together, the drug layer is sandwiched between the base plate and the sandwiching body in a portion in which the base plate and the sandwiching body are not attached together, and the protection body is attached to a region extending from the other end of the base plate to at least a halfway portion in a longitudinal direction of the sandwiching body.

14. The drug support body according to claim 12 or 13,
wherein the sandwiching body extends over a region between an end portion at the one end of the base plate and the fragile portion, and the protection body extends from an end portion at the other end of the base plate to the halfway portion of the sandwiching body.

15. The drug support body according to any one of claims 12 to 14,
wherein a non-attached portion to which the sandwiching body is not attached is formed in a predetermined region extending from an end portion of the protection body on the sandwiching body side.
